(19)

**Europäisches Patentamt**
**European Patent Office**
**Office européen des brevets**

(11) **EP 1 928 318 B1**

(12) **EUROPEAN PATENT SPECIFICATION**

(45) Date of publication and mention
of the grant of the patent:
**23.01.2013 Bulletin 2013/04**

(21) Application number: **06796000.5**

(22) Date of filing: **12.09.2006**

(51) Int Cl.:
**A61B 6/03** (2006.01)

(86) International application number:
**PCT/IB2006/053227**

(87) International publication number:
**WO 2007/034359 (29.03.2007 Gazette 2007/13)**

(54) **QUANTITATIVE MATERIAL DECOMPOSITION FOR SPECTRAL CT**

QUANTITATIVE MATERIALZERSETZUNG FÜR SPEKTRALE COMPUTERTOMOGRAPHIE

DECOMPOSITION MATERIELLE QUANTITATIVE POUR SCANOGRAPHIE SPECTRALE

(84) Designated Contracting States:
**AT BE BG CH CY CZ DE DK EE ES FI FR GB GR HU IE IS IT LI LT LU LV MC NL PL PT RO SE SI SK TR**

(30) Priority: **22.09.2005 EP 05108746**

(43) Date of publication of application:
**11.06.2008 Bulletin 2008/24**

(73) Proprietors:
• **Philips Intellectual Property & Standards GmbH**
**20099 Hamburg (DE)**
Designated Contracting States:
**DE**
• **Koninklijke Philips Electronics N.V.**
**5621 BA Eindhoven (NL)**
Designated Contracting States:
**AT BE BG CH CY CZ DK EE ES FI FR GB GR HU IE IS IT LI LT LU LV MC NL PL PT RO SE SI SK TR**

(72) Inventor: **PROKSA, Roland**
**52066 Aachen (DE)**

(74) Representative: **Verweij, Petronella Danielle et al**
**Philips Intellectual Property & Standards**
**P.O. Box 220**
**5600 AE Eindhoven (NL)**

(56) References cited:
**US-A1- 2004 101 088     US-A1- 2004 264 628**

• **SUKOVIC P AND CLINTHORNE N H ED - PIURI V ET AL:** "Basis Material Decomposition Using Triple-Energy X-ray Computed Tompgraphy" **INSTRUMENTATION AND MEASUREMENT TECHNOLOGY CONFERENCE, 1999. IMTC/99. PROCEEDINGS OF THE 16TH IEEE VENICE, ITALY 24-26 MAY 1999, PISCATAWAY, NJ, USA, IEEE, US, vol. 3, 24 May 1999 (1999-05-24), pages 1615-1618, XP002478941 ISBN: 978-0-7803-5276-6**
• **ARFELLI F:** "Synchrotron light and imaging systems for medical radiology" **NUCLEAR INSTRUMENTS & METHODS IN PHYSICS RESEARCH, SECTION - A:ACCELERATORS, SPECTROMETERS, DETECTORS AND ASSOCIATED EQUIPMENT, ELSEVIER, AMSTERDAM, NL, vol. 454, no. 1, 1 November 2000 (2000-11-01), pages 11-25, XP004238219 ISSN: 0168-9002**
• **FIRSCHING M ET AL:** "A method for stoichiometric material reconstruction with spectroscopic X-ray pixel detectors" **NUCLEAR SCIENCE SYMPOSIUM CONFERENCE RECORD, 2004 IEEE ROME, ITALY 16-22 OCT. 2004, PISCATAWAY, NJ, USA,IEEE, vol. 7, 16 October 2004 (2004-10-16), pages 4116-4119, XP010819606 ISBN: 978-0-7803-8700-3**
• **JANOS SWANPALMER ET AL:** "The feasibility of triple-energy absorptiometry for the determination of bone mineral, Ca and P in vivo" **PHYSIOLOGICAL MEASUREMENT, INSTITUTE OF PHYSICS PUBLISHING, BRISTOL, GB, vol. 19, no. 1, 1 February 1998 (1998-02-01), pages 1-15, XP020073818 ISSN: 0967-3334**

**Description**

The present invention relates to a CT system for determining the quantitative material concentrations of the components in a region of interest of an object with known Compton attenuation coefficients and photoelectric attenuation coefficients for said components. Further the present invention relates to a data processing device for use in such a CT system and to a corresponding data processing method. Still further, the present invention relates to a computer program for implementing said data processing method on a computer.

To provide quantitative material decomposition has already been an object of CT technology (computered tomography technology) in the past. A well-known method for so-called dual energy analysis is the subtraction (or division) of images acquired at different energies to enhance the contrast. However, the method is heuristic and a physical or biological meaning of the images is not defined. Furthermore, dual energy CT is capable to present two images with equivalent material compositions. An often used decompositon uses soft tissue and bone equivalence, like it is, for instance, described in Kalender, W.A. et al., "Evaluation of a Prototype Dual Energy Computer Tomographic Apparatus. I. Phantom Studies", Medical physics, Vol. 13, No. 3, May/June 1986, pages 334 to 339. However, a high value in the bone image does not mean that there is really bone. It only means that the presented combination of bone and soft tissue would yield the same measurements.

The explanatory power of such results is marginal since this method leaves a wide range of interpretations.

US 2004/0101088 A1 discloses a method and apparatus for discriminating between two different contrast agents or for discriminating between a contrast agent and an interventional tool. For this purpose, a multi-energy computer tomography (MECT) system is used. The MECT system includes a radiation source, a radiation detector and a computer coupled to the radiation source and to the radiation detector. The MECT system uses a decomposition algorithm, for example a Compton and photoelectric decomposition algorithm. The MECT system conducts pre-processing and reconstructing low energy projection data and high energy projection data to generate a low energy image and a high energy image. The high energy image and the low energy image are decomposed using the Compton and photoelectric decomposition algorithm to generate a first density map and a second density map. In case between two contrast agents shall be discriminated the first density map represents the first contrast agent and the second density map represents the second contrast agent. In case between a contrast agent and an interventional tool shall be discriminated the first density map represents the contrast agent and the second density map represents the interventional tool. In an alternative embodiment, the low energy projection data and the high energy projection data are decomposed prior to reconstructing first density map and second density map. The first density map and the second density map are displayed on a display using the computer. Based on the Compton and photoelectric decomposition algorithm, with this method and apparatus solely two density maps can be generated and therefore solely two contrast agents or solely one contrast agent and one interventional tool can be resolved.

P. Sukovic et al.: "Basis Material Decomposition Using Triple-Energy X-ray Computer Tomography", Proceedings of the 16th IEEE Instrumentation and Measurement Technology Conference, 1999. IMTC/99, vol. 3, 1999, pages 1615-1618, ISBN 0-7803-5276-9 represents a triple-energy reconstruction method in an X-ray tomography. The object to be examined is composed of three basis materials, namely soft tissue, bone and a contrast agent with a k-edge, particularly Gadolinium. For resolving these three basis materials the attenuation coefficient being a function of energy is modeled as a linear combination of three basis functions. A first basis function approximates the energy dependence of the photoelectric interactions, a second basis function approximates the energy dependence of the total cross-section for Compton scattering and a third basis function represents energy dependence of the attenuation coefficient of the contrast agent Gadolinium. Knowing the mass-attenuation coefficients of the three basis materials the basis function based linear combination is rewritten as a linear combination existing of three products, each product representing one basis material and existing of a mass-attenuation coefficient of the respective basis material and the density of this basis material. The densities of the three basis materials are determined by solving the density based linear combination and are used for displaying purposes. With this approach being based on the Compton effect, the photoelectric effect and the k-edge effect solely three basis materials can be resolved.

US 2004/0264628 A1 discloses a multi-spectral X-ray imaging system using a wideband source and a filtration assembly to select for sets of spectral data. This system can be used in CT imaging. Processed data, such as a CT reconstructed image, is decomposed onto basis functions for analytical processing of multi-spectral image data to facilitate computer assisted diagnostics. The total linear attenuation coefficient is modeled as a linear combination of three basis functions. A first basis function represents photoelectric absorption, a second basis function represents Compton scattering and a third basis function represents Rayleigh scattering. A stack of multi-spectral or hyper-spectral co-registered images is decomposed onto multiple basis function images. The multi-basis function images form an input to a generalized computer assisted diagnosis (CAD) algorithm. A Bayesian model is introduced that assumes the result of known CAD algorithms working on each basis function. This model integrates the likelihood that for each basis function the image contains a feature of interest, such as cancer in medical imaging. Therefore, with three basis functions solely three different materials can be resolved.

Thus, there is a lack of proper techniques for quantitative material decomposition which provide adequate results.

It is thus an object of the present invention to provide a CT system which improves the quality and explanatory power of quantitative material decomposition but which is still kept simple and thus easy to handle. Further, a corresponding data processing device and data processing method shall be provided.

The object is achieved according to the present invention by a CT system as defined in claim 1 comprising:

- a scanning unit having a radiation source and a detector unit for acquisition of spectral CT projection data from said region of interest,
- a modeling unit for obtaining a photoelectric effect projection data set and a Compton effect projection data set by decomposing said spectral CT projection data set by means of respective models of photoelectric effect and Compton effect,
- a reconstruction unit for reconstructing a photoelectric effect image and a Compton effect image of said region of interest from said photoelectric effect projection data set and Compton effect projection data set,
- a processing unit for determining the concentrations of said components in said region of interest by solving a system of equations obtained by equating said photoelectric effect image data with the accumulated products of said concentrations and photoelectric attenuation coefficients for said components and equating said Compton effect image data with the accumulated products of said concentrations and Compton attenuation coefficients for said components for obtaining a first concentration of a first component and a second concentration of a second component and by subtracting the first concentration and the second concentration from 1 for obtaining a third concentration of a third component.

An appropriate data processing device for use in such a CT system and a corresponding data processing method are defined in claims 12 and 13. A computer program which may be stored on a record carrier for implementing said data processing method on a computer is defined in claim 14. Preferred embodiments of the invention are defined in the dependent claims.

The present invention is based on the idea to use spectral CT data for quantitative material decomposition and further on the idea, that a number m of measurable physical effects in a region of interest allows to determine the concentrations of (m + 1) components in said region of interest. It is assumed that a spectral CT scanner acquires projection data with different spectral coding. These data can be fitted to a physical model of the object. The physical model contains several physical effects such as Compton effect, photoelectric effect and K-edge effect. If K-edges are identified, the related decomposition is directly achieved by dividing the physical model of the objects into the physical models of the effects. The two model parameters of the remaining effects (Compton effect and photoelectric effect) allow to decompose a mixture of the remaining at most three components. Any ingredient i with density $p_i$, atomic weight $A_i$ and atomic number $Z_i$ has Compton and photoelectric attenuation of

$$\mu_i \text{compton}(E) = K_1 \rho_i A_i{-}1 Z_i\, f_{KN}(E)$$

$$\mu_i \text{photo}(E) = K_2 \rho_i A_i{-}1 Z_i^n E^{-3}.$$

K1 and K2 are constant, n has a value of about 4, and $f_{KN}(E)$ is the so-called integrated Klein-Nishina function. The engery independend attenuation contributions are referred to as $\eta_i$compton and $\eta_i$photo, hereinafter, with:

$$\eta_i \text{compton} = \rho_i A_i{-}1 Z_i$$

$$\eta_i \text{photo} = \rho_i A_i{-}1 Z_i^n.$$

If there are three ingredients with relative volumetric concentrations $c_1$, $c_2$ and $c_3 = (1{-}c_1{-}c_2)$ the following system of equations is obtained:

$$\eta_{mixture}^{compton} = c_1 \eta_1^{compton} + c_2 \eta_2^{compton} + (1 - c_1 - c_2)\eta_3^{compton}$$

$$\eta_{mixture}^{photo} = c_1 \eta_1^{photo} + c_2 \eta_2^{photo} + (1 - c_1 - c_2)\eta_3^{photo} \, .$$

This system of equations can be solved to the two unknowns ($c_1$ and $c_2$) on a per voxel basis or with at least $\chi 2$ fit over a region of interest. The materials whose concentrations are to be determined can be elements, molecules or any known mixture.

In a preferred embodiment the number of the components whose concentrations are to be determined is restricted to at most three. Thus, the determination of the concentrations of the components is easier since only two physical effects have to be considered. However, in another embodiment the quantitative relative material concentrations of four components in the region of interest are determined wherein for at least one of said components the K-edge effect is known and wherein said modeling unit is adapted for additionally obtaining a K-edge projection data set by using a respective model of K-edge effect corresponding to said at least one component and thereby indicating the concentration of said at least one component. Thus, it is possible to consider an additional component. Advantageously, the number of said components is restricted to at most four.

Advantageously, said reconstruction unit performs an additional reconstruction by calculating the fourth concentration of said at least one component, wherein in addition to said photoelectric effect image and said Compton effect image said fourth concentration is provided to said processing unit. This is a very simple implementation leading to a CT system being capable of resolving four different materials.

Advantageously, said processing unit is adapted for determining said third concentration by subtracting the first concentration, the second concentration and the fourth concentration from 1. Thus, the determination of the concentrations of the components is easier since only three physical effects have to be considered. Advantageously, said photoelectric effect projection data set and said Compton effect projection data set derived by said modelling unit are energy independent quantities.

Advantageously, said at least one component with known K-edge effect is indicated by a contrast agent, particularly Iodine or Gadolinium, which is injected into said object. These contrast agents feature readily identifiable K-edge effects and thus facilitate the determination of the concentration of said additional component.

Additionally, the invention can be used to measure a "spectral footprint" of unknown mixtures that can be further used for improved analysis. If in an example with a contrast agent (CA) it is possible to define a region that contains only CA and one another component such as blood, e.g. a chamber, it can be decomposed into the component (blood) and CA and allows a quantitative measure of the CA concentration. The mixture (blood, CA) can be used as a spectral footprint for the decomposition of other areas. The other area can be decomposed into blood-CA mixture, soft tissue and calcium or into blood-CA mixture, soft tissue and metal for example. The quality of the decomposition depends on the dissimilarity of the spectral footprint of the components and improves if the density and/or the atomic number differences are large. By measuring a "spectral footprint" the invention offers more flexibility and allows to determine the concentrations of the components even if there are more than three components/subcomponents.

In an embodiment of the present invention at least one of said components (referred to as a compound component, hereinafter) is represented by one or more subcomponents. Thus, a compound component may contain a mixture of subcomponents, for example, in case that Compton attenuation coefficients and photoelectric attenuation coefficients are only known for the mixture and not for each subcomponent. This also allows to determine the concentration of a compound component in a region of interest if the total number of ingredients in a region of interest in the form of subcomponents is not restricted to three, in case only Compton attenuation coefficients and photoelectric attenuation coefficients are known, or is not restricted to four, in case for at least one of said components the K-edge effect is known. Advantageously said known Compton attenuation coefficients and photoelectric attenuation coefficients of said compound component represented by one or more subcomponents are obtained by said modeling unit as said photoelectric effect projection data set and said Compton effect projection data set, respectively. Thus, even if the Compton attenuation coefficient and the photoelectric attenuation coefficient of said at least one compound component represented by one or more subcomponents are unknown the invention allows to determine the concentration of the components in a region of interest by determining the Compton attenuation coefficient and the photoelectric attenuation coefficient of said compound component.

The invention will now be described in more detail by way of embodiments with reference to the drawings in which

Fig. 1 shows a block diagram of a CT system according to the invention,
Fig. 2 shows a flowchart of a data processing method according to the invention,

Fig. 3 shows a graph representing the attenuation spectrum of Gadolinium.

The first embodiment describes a device and a method for determining the quantitative material concentrations of three components in a region of interest of an object. Compton attenuation coefficients and photoelectric attenuation coefficients for said components are known.

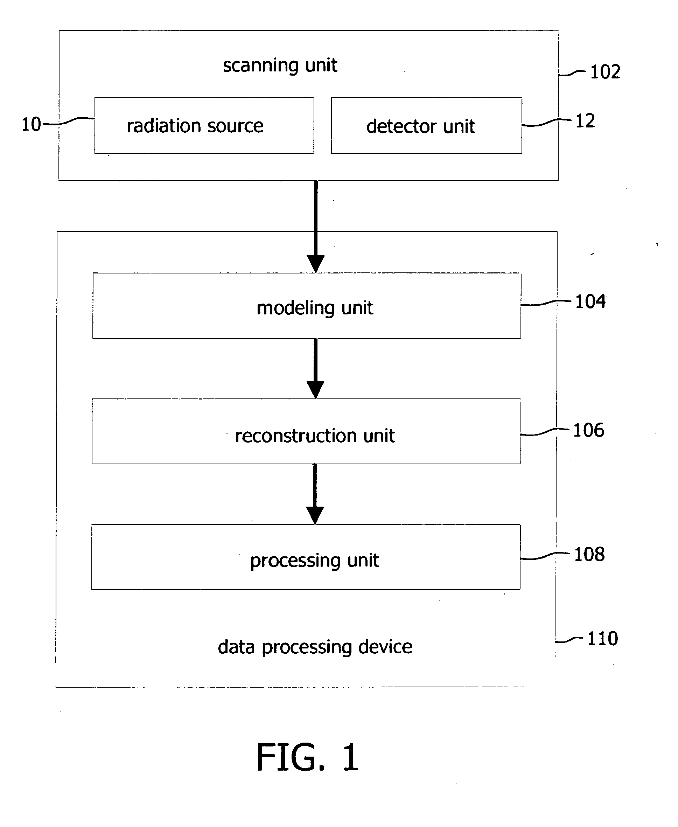

Fig. 1 shows a spectral CT system according to the first embodiment comprising a scanning unit 102, a modeling unit 104, a reconstruction unit 106 and a processing unit 108. Said scanning unit 102 comprises a radiation source 10 and a detector unit 12. Said modeling unit, said reconstruction unit 106 and said processing unit 108 compose a data processing device 110.

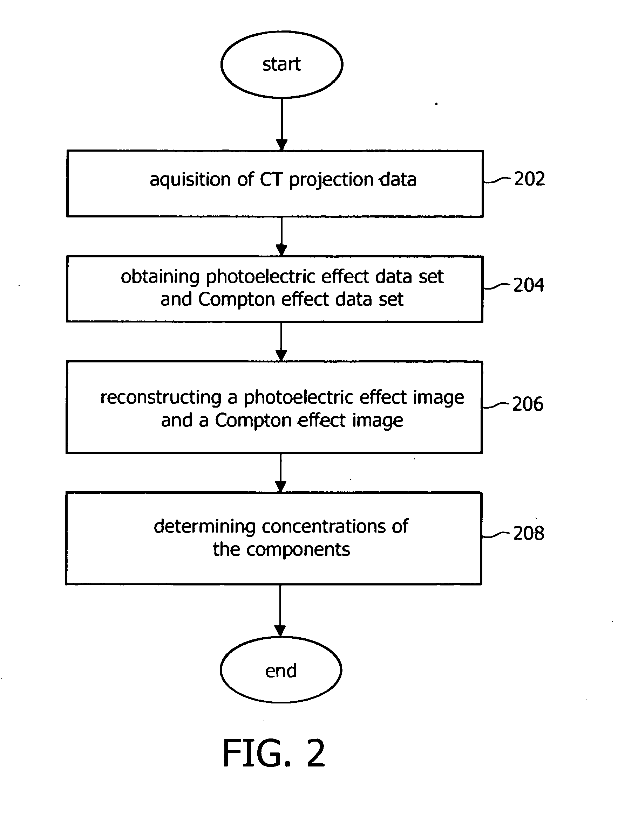

As shown in Fig. 2 in a first step 202 said scanning unit 102 acquires spectral CT projection data of an object or of a region of interest of said object. To achieve this, said radiation source 10 of said scanning unit 102 emits a radiation beam of spectral energy towards said detector unit 12. Said detector unit 12 receives the respective energy signals diminished by the attenuation caused during the passage of the radiation beam through the object. In this first embodiment the detector unit 12 is constituted by an energy-resolving X-ray detector. Such energy-resolving X-ray detector may be available in the near future. It is also possible to use a conventional dual layer detector or a dual energy scanner. Said energy signals acquired by the detector unit 12 as said spectral CT projection data is provided to said modeling unit 104.

In a second step 204 said modeling unit 104 obtains a photoelectric effect projection data set and a Compton effect projection data set which correspond to the spatial distribution of the attenuation caused by photoelectric effect and Compton effect, respectively. Said photoelectric effect projection data set and said Compton effect projection data set are obtained by decomposing said spectral CT projection data set acquired by said step 202 with scanning unit 102. A typical physical model which includes the measurement has the form

$$M_j \approx \int S_j(E) e^{-\int_R (\mu^{photo}(x,E) + \mu^{compton}(x,E)) ds} \, dE$$

where $M_j$ is the result of measurement j, acquired with a scanning unit channel that has the spectral sensitivity $S_j(E)$. The measurement was performed along a ray R on which the energy (E) and spatial (x) dependent linear attenuations $\mu^{photo}(x, E)$, $\mu^{compton}(x, E)$ were integrated (ds). The modeling unit 104 derives the energy independend quantities

$$\int_R \eta^{photo}(x) ds \text{ and } \int_R \eta^{compton}(x) ds \,.$$

In a third step 206 said reconstruction unit 106 reconstructs two images $\eta^{photo}(x)$ and $\eta^{compton}(x)$ from the calculated line integrals $\int \eta^{photo}(x) ds$ and $\int \eta^{compton}(x) ds$. R R

In a fourth step 208 said processing unit 108 determines the concentrations of said three components in said region of interest. This is achieved by solving the following system of equations:

$$\eta_{mixture}^{compton}(x) = c_1(x)\eta_1^{compton}(x) + c_2(x)\eta_2^{compton}(x) + (1 - c_1(x) - c_2(x))\eta_3^{compton}(x)$$

$$\eta_{mixture}^{photo}(x) = c_1(x)\eta_1^{photo}(x) + c_2(x)\eta_2^{photo}(x) + (1 - c_1(x) - c_2(x))\eta_3^{photo}(x) \,.$$

$\eta^{Compton}(x)$ and $\eta^{photo}(x)$ correspond as above to said photoelectric effect projection data set and Compton effect projection data set, which correspond to the spatial distribution of the attenuation caused by photoelectric effect and Compton effect. $c_1(x)$ and $c_2(x)$ represent the concentrations of component 1 and component 2 at point x to be determined, respectively. A concentration $c_3(x)$ of a third component 3 is obtained by subtracting $c_1(x)$ and $c_2(x)$ from 1 ($c_3(x) = 1-c_1(x) - c_2(x)$) since there are only three components in the region of interest. $\eta_1^{Compton}(x)$, $\eta_2^{Compton}(x)$ and $\eta_3^{Compton}(x)$ correspond to the Compton attenuation coefficients of said components, respectively, which are known and can, for example, be acquired by so-called NIST tables. $\eta_1^{photo}(x)$, $\eta_2^{photo}(x)$ and $\eta_3^{photo}(x)$ correspond to the photoelectric attenuation coefficients of said components, respectively, which are known as well and can also, for example, be obtained by so-called NIST (National Institute of Standards and Technology) tables. The above system of equations can be solved to the two unknowns ($c_1(x)$ and $c_2(x)$), and $c_3(x)$ can be determined therefrom. Said concentrations $c_1(x)$, $c_2(x)$, $c_3(x)$ of said three components are obtained on a per voxel basis or with a least squares fit over the region of interest.

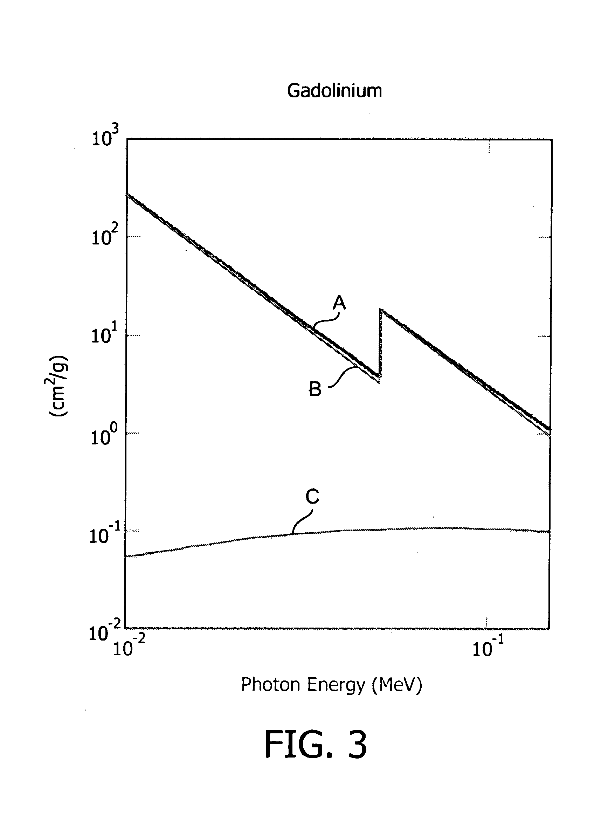

A second embodiment describes a spectral CT system for determining the quantitative material concentrations of four components in a region of interest of an object with known Compton attenuation coefficients and photo electric attenuation coefficients for said components and with known K-edge effect for at least one of said components. In this second embodiment said at least one component with known K-edge effect is Gadolinium with a K-edge effect at about 60 keV. A graph representing the attentuation spectrum of Gadolinium is shown in Fig. 3 with attentuation (1/cm) per mass density (g/cm3). Fig. 3 shows the total attenuation with coherent scattering A, the attenuation caused by the photo electric effect B, and the attenuation caused by Compton effect (incoherent scattering) C. But any other material with a K-edge effect which is applicable to differ from other materials in the scanned object may be used. This may be, for example, any other contrast agent, such as, for example, Iodine which has a K-edge effect at about 30 keV.

The second embodiment differs from the first embodiment in that in said second step 204 said modeling unit 104 is adapted to additionally obtain a K-edge projection data set corresponding to the attenuation caused by K-edge effect. Said K-edge projection data set is obtained by decomposing said spectral CT projection data acquired by said scanning unit 102 by means of respective models of photoelectric effect, Compton effect and K-edge effect, as follows:

$$\mu^{\text{total}}(E) = \mu^{\text{photo}}(E) + \mu^{\text{compton}}(E) + \mu^{\text{K-edge}}(E).$$

The above-mentioned typical physical model is extended with the related absorption of the contrast agent

$$M_j \approx \int S_j(E) e^{-\int_R (\mu^{photo}(x,E) + \mu^{compton}(x,E) + c_4(x)\mu^{K-edge}(E))ds} \, dE$$

A typical spectrum of Gadolinium $\mu^{\text{K-edge}}(E)$ is shown in Fig. 3. It is given in arbsorption per mass density. The relative concentration of Gadolinium $c_4(x)$ in a mixture becomes the third model parameter that is calulated by the modeling unit 104. As for the photo and Compton effect projection data, the result is given by a line integral $\int_R c_4(x)ds$.

The R reconstruction unit 106 performs an additional reconstruction by calculating $c_4(x)$. Said photoelectric effect image, said Compton effect image and said concentration $c_4(x)$ of Gadolinium, i.e. said fourth component, are provided to said processing unit 108.

Further in this second embodiment in said fourth step 208 said processing unit 108 is adapted to determine the concentrations of the remaining three components in said region of interest. This is achieved by solving the following system of equations:

$$\eta^{compton}(x) = c_1(x)\eta_1^{compton}(x) + c_2(x)\eta_2^{compton}(x) + (1 - c_1(x) - c_2(x) - c_4(x))\eta_3^{compton}(x)$$

$$\eta^{photo}(x) = c_1(x)\eta_1^{photo}(x) + c_2(x)\eta_2^{photo}(x) + (1 - c_1(x) - c_2(x) - c_4(x))\eta_3^{photo}(x).$$

Same variables denote same terms as in embodiment 1. Additionally, $c_4(x)$ represents the concentration of said fourth component. A concentration $c_3(x)$ of a third component 3 is obtained by subtracting $c_1(x)$, $c_2(x)$ and $c_4(x)$ from 1 ($c_3(x) = 1 - c_1(x) - c_2(x) - c_4(x)$) since there are only four components in the region of interest. The above system of equations can be solved to the two unknowns ($c_1(x)$ and $c_2(x)$) and $c_3(x)$ can be determined therefrom. Said concentrations $c_1(x)$, $c_2(x)$, $c(x)_3$ and $c_4(x)$ of said four components are obtained on a per voxel basis or with a least squares fit over the region of interest.

A third embodiment describes a CT system for determing a so-called spectral footprint of a component (referred to as a compound component, hereinafter) represented by one or more subcomponents, e.g. elements, molecules or a mixture. A subcomponent means a material, for which at least photoelectric attenuation coefficient and Compton attenuation coefficient are to be determined for any reason, for example, because neither photoelectric attenuation coefficient nor Compton attenuation coefficient nor K-edge effect are known for a material or for a mixture of materials. A spectral footprint means the photoelectric attenuation coefficient and Compton attenuation coefficient of said compound component determined by said CT system before determining the concentrations of the components.

The third embodiment can be realized in two different ways. The first way is to use said CT system to image a sample of the component. In this case, the reconstructed images $\eta^{photo}(x)$ and $\eta^{compton}(x)$ represent the spectral footprint of the compound component and can be used in further examinations to represent one of three or four components, respectively. The second way is to select a region of interest in the reconstruction images that contains a homogenious distribution of the compound component. The mean value of the images $\eta^{photo}(x)$ and $\eta^{compton}(x)$ in this area can be used as the spectral footprint for the analysis in other areas.

It is possible to combine the features of above embodiments and in this way to determine the concentrations of plural components and/or to determine spectral footprints of plural components at once in case the concentrations of said components are known. For example embodiment 3 and embodiment 2 can be combined to determine a spectral footprint of an unknown component in case there is an unknown component and a component with known photoelectric attenuation coefficient, Compton attenuation coeffecient and K-edge effect, such as a CA, in a region of interest.

**Claims**

1. CT system for determining the quantitative relative material concentrations of the components in a region of interest of an object with known Compton attenuation coefficients and photoelectric attenuation coefficients for said components comprising:

   - a scanning unit (102) having a radiation source (10) and a detector unit (12) for acquisition of spectral CT projection data from said region of interest;
   - a modeling unit (104) for obtaining a photoelectric effect projection data set and a Compton effect projection data set by decomposing said spectral CT projection data set by means of respective models of photoelectric effect and Compton effect;
   - a reconstruction unit (106) for reconstructing a photoelectric effect image and a Compton effect image of said region of interest from said photoelectric effect projection data set and Compton effect projection data set;
   - a processing unit (108) for determining the concentrations of said components in said region of interest by solving a system of equations obtained by equating said photoelectric effect image data with the accumulated products of said concentrations and photoelectric attenuation coefficients for said components and equating said Compton effect image data with the accumulated products of said concentrations and Compton attenuation coefficients for said components for obtaining a first concentration ($c_1(x)$) of a first component and a second concentration ($c_2(x)$) of a second component and by subtracting the first concentration ($c_1(x)$) and the second concentration ($c_2(x)$) from 1 for obtaining a third concentration ($c_3(x)$) of a third component.

2. CT system as claimed in claim 1,
   wherein the number of said components is restricted to at most three.

3. CT system as claimed in claim 1,
   wherein the quantitative relative material concentrations of four components in the region of interest with known K-edge effect for at least one of said components are determined and
   wherein said modeling unit is adapted for additionally obtaining a K-edge projection data set by using a respective model of K-edge effect corresponding to said at least one component and thereby indicating the concentration of said at least one component.

4. CT system as claimed in claim 3,
   wherein the number of said components is restricted to at most four.

5. CT system as claimed in claim 3,
   wherein said reconstruction unit (106) performs an additional reconstruction by calculating the fourth concentration ($c_4(x)$) of said at least one component,
   wherein in addition to said photoelectric effect image and said Compton effect image said fourth concentration ($c_4(x)$) is provided to said processing unit (108).

6. CT system as claimed in claim 5,
wherein said processing unit (108) is adapted for determining said third concentration ($c_3(x)$) by subtracting the first concentration ($c_1(x)$), the second concentration ($c_2(x)$) and the fourth concentration ($c_4(x)$) from 1.

7. CT system as claimed in claim 1,
wherein said photoelectric effect projection data set and said Compton effect projection data set derived by said modelling unit (104) are energy independent quantities.

8. CT system as claimed in claim 3,
wherein said at least one component is indicated by a contrast agent injected into said object.

9. CT system as claimed in claim 8,
wherein said contrast agent is Iodine or Gadolinium.

10. CT system as claimed in claim 1 to 9,
wherein at least one of said components is represented by one or more subcomponents.

11. CT system as claimed in claim 10,
wherein said known Compton attenuation coefficients and photoelectric attenuation coefficients of said at least one component are obtained by said modeling unit as said photoelectric effect projection data set and said Compton effect projection data set, respectively.

12. Data processing device (110) for use in a CT system for determining the quantitative relative material concentrations of the components in a region of interest of an object with known Compton attenuation coefficients and photoelectric attenuation coefficients for said components, said data processing device being provided with spectral CT projection data of said region of interest, said spectral CT projection data being obtained from a scanning unit (102), comprising:

- a modeling unit (104) for obtaining a photoelectric effect projection data set and a Compton effect projection data set by decomposing said spectral CT projection data set by means of respective models of photoelectric effect and Compton effect;
- a reconstruction unit (106) for reconstructing a photoelectric effect image and a Compton effect image of said region of interest from said photoelectric effect projection data set and Compton effect projection data set;
- a processing unit (108) for determining the concentrations of said components in said region of interest by solving a system of equations obtained by equating said photoelectric effect image data with the accumulated products of said concentrations and photoelectric attenuation coefficients for said components and equating said Compton effect image data with the accumulated products of said concentrations and Compton attenuation coefficients for said components for obtaining a first concentration ($c_1(x)$) of a first component and a second concentration ($c_2(x)$) of a second component and by subtracting the first concentration ($c_1(x)$) and the second concentration ($c_2(x)$) from 1 for obtaining a third concentration ($c_3(x)$) of a third component.

13. Data processing method for use in a CT system for determining the quantitative relative material concentrations of the components in a region of interest of an object with known Compton attenuation coefficients and photoelectric attenuation coefficients for said components, said data processing method being provided with CT projection data of said region of interest, said CT projection data being obtained from a scanning unit, comprising the steps of:

- obtaining (204) a photoelectric effect projection data set and a Compton effect projection data set by decomposing said spectral CT projection data set by means of respective models of photoelectric effect and Compton effect;
- reconstructing (206) a photoelectric effect image and a Compton effect image of said region of interest from said photoelectric effect projection data set and Compton effect projection data set;
- determining (208) the concentrations of said components in said region of interest by solving a system of equations obtained by equating said photoelectric effect image data with the accumulated products of said concentrations and photoelectric attenuation coefficients for said components and equating said Compton effect image data with the accumulated products of said concentrations and Compton attenuation coefficients for said components for obtaining a first concentration ($c_1(x)$) of a first component and a second concentration ($c_2(x)$) of a second component and by subtracting the first concentration ($c_1(x)$) and the second concentration ($c_2(x)$) from 1 for obtaining a third concentration ($c_3(x)$) of a third component.

14. Computer program comprising computer program code means for causing a computer to perform the steps of the method as claimed in claim 13 when said computer program is run on a computer.

**Patentansprüche**

1. CT-System zur Bestimmung der quantitativen relativen Materialkonzentrationen der Bestandteile in einer interessierenden Region eines Objekts mit bekannten Compton-Schwächungskoeffizienten und Photoschwächungskoeffizienten für die genannten Bestandteile, das Folgendes umfasst:

   - einen Scanner (102) mit einer Strahlungsquelle (10) und einer Detektoreinheit (12) zum Erfassen von Spektral-CT-Projektionsdaten aus der genannten interessierenden Region,
   - eine Modelliereinheit (104) zum Erzielen eines Satzes mit Photoeffekt-Projektionsdaten und eines Satzes mit Compton-Effekt-Projektionsdaten durch Zerlegung des genannten Satzes mit Spektral-CT-Projektionsdaten mit Hilfe entsprechender Modelle des Photoeffekts und des Compton-Effektes,
   - eine Rekonstruktionseinheit (106) zum Rekonstruieren eines Photoeffekt-Bildes und eines Compton-Effekt-Bildes der genannten interessierenden Region aus dem genannten Satz mit Photoeffekt-Projektionsdaten und dem genannten Satz mit Compton-Effekt-Projektionsdaten,
   - eine Prozessoreinheit (108) zum Bestimmen der Konzentrationen der genannten Bestandteile in der genannten interessierenden Region durch Lösung eines Gleichungssystems, das durch das Gleichsetzen der genannten Photoeffekt-Bilddaten mit den akkumulierten Produkten aus den genannten Konzentrationen und den Photoschwächungskoeffizienten für die genannten Bestandteile und das Gleichsetzen der genannten Compton-Effekt-Bilddaten mit den akkumulierten Produkten aus den genannten Konzentrationen und den Compton-Schwächungskoeffizienten für die genannten Bestandteile erzielt wird, um eine erste Konzentration ($c_1(x)$) eines ersten Bestandteils und eine zweiten Konzentration ($c_2(x)$) eines zweiten Bestandteils zu erhalten, und durch Subtraktion der ersten Konzentration ($c_1(x)$) und der zweiten Konzentration ($c_2(x)$) von 1, um eine dritte Konzentration ($c_3(x)$) eines dritten Bestandteils zu erhalten.

2. CT-System nach Anspruch 1,
   wobei die Anzahl der genannten Bestandteile auf höchstens drei begrenzt ist.

3. CT-System nach Anspruch 1,
   wobei die quantitativen relativen Materialkonzentrationen von vier Bestandteilen in der interessierenden Region mit dem bekannten K-Kanteneffekt für mindestens einen der genannten Bestandteile bestimmt werden und
   wobei die genannte Modelliereinheit so ausgelegt ist, dass sie zusätzlich einen Satz mit K-Kanten-Projektionsdaten erzielt, indem sie ein entsprechendes Modell des K-Kanteneffekts verwendet, das dem genannten mindestens einen Bestandteil entspricht und dadurch die Konzentration des genannten mindestens einen Bestandteils angibt.

4. CT-System nach Anspruch 3,
   wobei die Anzahl der genannten Bestandteile auf höchstens vier begrenzt ist.

5. CT-System nach Anspruch 3,
   wobei die genannte Rekonstruktionseinheit (106) eine zusätzliche Rekonstruktion durchführt, indem sie die vierte Konzentration ($c_4(x)$) des genannten mindestens einen Bestandteils berechnet,
   wobei zusätzlich zu dem genannten Photoeffekt-Bild und dem genannten Compton-Effekt-Bild der genannten Prozessoreinheit (108) die genannte vierte Konzentration ($c_4(x)$) übermittelt wird.

6. CT-System nach Anspruch 5,
   wobei die genannte Prozessoreinheit (108) so ausgelegt ist, dass sie die genannte dritte Konzentration ($c_3(x)$) durch Subtraktion der ersten Konzentration ($c_1(x)$), der zweiten Konzentration ($c_2(x)$) und der vierten Konzentration ($c_4(x)$) von 1 bestimmt.

7. CT-System nach Anspruch 1,
   wobei der genannte Satz mit Photoeffekt-Projektionsdaten und der genannte Satz mit Compton-Effekt-Projektionsdaten, die von der genannten Modelliereinheit (104) abgeleitet wurden, energieunabhängige Größen sind.

8. CT-System nach Anspruch 3, wobei der genannte mindestens eine Bestandteil durch ein in das genannte Objekt injizierte Kontrastmittel angezeigt wird.

9. CT-System nach Anspruch 8, wobei das genannte Kontrastmittel Jod oder Gadolinium ist.

10. CT-System nach den Ansprüchen 1 bis 9,
wobei mindestens einer der genannten Bestandteile durch eine oder mehrere Teilbestandteile dargestellt wird.

11. CT-System nach Anspruch 10,
wobei die genannten bekannten Compton-Schwächungskoeffizienten und Photoschwächungskoeffizienten des genannten mindestens einen Bestandteils durch die genannte Modelliereinheit als der genannte Satz mit Photoeffekt-Projektionsdaten bzw. als der genannte Satz mit Compton-Effekt-Projektionsdaten erzielt werden.

12. Datenprozessor (110) zur Verwendung in einem CT-System zur Bestimmung der quantitativen relativen Materialkonzentrationen der Bestandteile in einer interessierenden Region eines Objekts mit bekannten Compton-Schwächungskoeffizienten und Photoschwächungskoeffizienten für die genannten Bestandteile, wobei dem genannten Datenprozessor Spektral-CT-Projektionsdaten der genannten interessierenden Region übermittelt werden, wobei die genannten Spektral-CT-Projektionsdaten von einem Scanner (102) erzielt werden, wobei der Datenprozessor Folgendes umfasst:

- eine Modelliereinheit (104) zum Erzielen eines Satzes mit Photoeffekt-Projektionsdaten und eines Satzes mit Compton-Effekt-Projektionsdaten durch Zerlegung des genannten Satzes mit Spektral-CT-Projektionsdaten mit Hilfe entsprechender Modelle des Photoeffekts und des Compton-Effektes,
- eine Rekonstruktionseinheit (106) zum Rekonstruieren eines Photoeffekt-Bildes und eines Compton-Effekt-Bildes der genannten interessierenden Region aus dem genannten Satz mit Photoeffekt-Projektionsdaten und dem genannten Satz mit Compton-Effekt-Projektionsdaten,
- eine Prozessoreinheit (108) zum Bestimmen der Konzentrationen der genannten Bestandteile in der genannten interessierenden Region durch Lösung eines Gleichungssystems, das durch das Gleichsetzen der genannten Photoeffekt-Bilddaten mit den akkumulierten Produkten aus den genannten Konzentrationen und den Photoschwächungskoeffizienten für die genannten Bestandteile und das Gleichsetzen der genannten Compton-Effekt-Bilddaten mit den akkumulierten Produkten aus den genannten Konzentrationen und den Compton-Schwächungskoeffizienten für die genannten Bestandteile erzielt wird, um eine erste Konzentration ($c_1(x)$) eines ersten Bestandteils und eine zweiten Konzentration ($c_2(x)$) eines zweiten Bestandteils zu erhalten, und durch Subtraktion der ersten Konzentration ($c_1(x)$) und der zweiten Konzentration ($c_2(x)$) von 1, um eine dritte Konzentration ($c_3(x)$) eines dritten Bestandteils zu erhalten.

13. Datenverarbeitungsverfahren zur Verwendung in einem CT-System zur Bestimmung der quantitativen relativen Materialkonzentrationen der Bestandteile in einer interessierenden Region eines Objekts mit bekannten Compton-Schwächungskoeffizienten und Photoschwächungskoeffizienten für die genannten Bestandteile, wobei dem genannten Datenprozessor Spektral-CT-Projektionsdaten der genannten interessierenden Region übermittelt werden, wobei die genannten Spektral-CT-Projektionsdaten von einem Scanner (102) erzielt werden, wobei das Verfahren folgende Schritte umfasst:

- Erzielen (204) eines Satzes mit Photoeffekt-Projektionsdaten und eines Satzes mit Compton-Effekt-Projektionsdaten durch Zerlegung des genannten Satzes mit Spektral-CT-Projektionsdaten mit Hilfe entsprechender Modelle des Photoeffekts und des Compton-Effektes,
- Rekonstruieren (206) eines Photoeffekt-Bildes und eines Compton-Effekt-Bildes der genannten interessierenden Region aus dem genannten Satz mit Photoeffekt-Projektionsdaten und dem genannten Satz mit Compton-Effekt-Projektionsdaten,
- Bestimmen (208) der Konzentrationen der genannten Bestandteile in der genannten interessierenden Region durch Lösung eines Gleichungssystems, das durch das Gleichsetzen der genannten Photoeffekt-Bilddaten mit den akkumulierten Produkten aus den genannten Konzentrationen und den Photoschwächungskoeffizienten für die genannten Bestandteile und das Gleichsetzen der genannten Compton-Effekt-Bilddaten mit den akkumulierten Produkten aus den genannten Konzentrationen und den Compton-Schwächungskoeffizienten für die genannten Bestandteile erzielt wird, um eine erste Konzentration ($c_1(x)$) eines ersten Bestandteils und eine zweiten Konzentration ($c_2(x)$) eines zweiten Bestandteils zu erhalten, und durch Subtraktion der ersten Konzentration ($c_1(x)$) und der zweiten Konzentration ($c_2(x)$) von 1, um eine dritte Konzentration ($c_3(x)$) eines dritten Bestandteils zu erhalten.

14. Computerprogramm, das Computerprogramm-Codemittel umfasst, die einen Computer veranlassen, die Schritte des Verfahrens nach Anspruch 13 auszuführen, wenn das genannte Computerprogramm auf einem Computer läuft.

**Revendications**

1. Système tomodensitométrique pour déterminer les concentrations relatives quantitatives de matériaux des composants dans une région d'intérêt d'un objet avec des coefficients d'atténuation de Compton et coefficients d'atténuation photoélectrique connus pour lesdits composants, comprenant :

- une unité tomographique (102) comportant une source de rayonnement (10) et une unité de détection (12) pour l'acquisition de données de projection tomodensitométriques spectrales à partir de ladite région d'intérêt ;
- une unité de modélisation (104) pour obtenir un jeu de données de projection d'effet photoélectrique et un jeu de données de projection d'effet de Compton en décomposant ledit jeu de données de projection tomodensitométriques spectrales au moyen de modèles respectifs d'effet photoélectrique et d'effet de Compton ;
- une unité de reconstruction (106) pour reconstruire une image à effet photoélectrique et une image à effet de Compton de ladite région d'intérêt à partir dudit jeu de données de projection d'effet photoélectrique et dudit jeu de données de projection d'effet de Compton ;
- une unité de traitement (108) pour déterminer les concentrations desdits composants dans ladite région d'intérêt en résolvant un système d'équations obtenues en associant lesdites données d'image à effet photoélectrique avec les produits accumulés desdites concentrations et desdits coefficients d'atténuation photoélectrique pour lesdits composants et en associant lesdites données d'image à effet de Compton avec les produits accumulés desdites concentrations et desdits coefficients d'atténuation de Compton pour lesdits composants pour obtenir une première concentration ($c_1(x)$) d'un premier composant et une deuxième concentration ($c_2(x)$) d'un deuxième composant et en soustrayant la première concentration ($c_1(x)$) et la deuxième concentration ($c_2(x)$) d'1 pour obtenir une troisième concentration ($c_3(x)$) d'un troisième composant.

2. Système tomodensitométrique selon la revendication 1, dans lequel le nombre desdits composants est limité à trois au maximum.

3. Système tomodensitométrique selon la revendication 1,
dans lequel les concentrations relatives quantitatives de matériaux de quatre composants dans la région d'intérêt avec un effet de seuil K connu pour au moins un desdits composants sont déterminées, et
dans lequel ladite unité de modélisation est adaptée pour obtenir en outre un jeu de données de projection de seuil K en utilisant un modèle respectif d'effet de seuil K correspondant audit au moins un composant et ainsi indiquer la concentration dudit au moins un composant.

4. Système tomodensitométrique selon la revendication 3, dans lequel le nombre desdits composants est limité à quatre au maximum.

5. Système tomodensitométrique selon la revendication 3,
dans lequel ladite unité de reconstruction (106) réalise une reconstruction supplémentaire en calculant la quatrième concentration ($c_4(x)$) dudit au moins un composant,
dans lequel, en plus de ladite image à effet photoélectrique et de ladite image à effet de Compton, ladite quatrième concentration ($c_4(x)$) est fournie à ladite unité de traitement (108).

6. Système tomodensitométrique selon la revendication 5,
dans lequel ladite unité de traitement (108) est adaptée pour déterminer ladite troisième concentration ($c_3(x)$) en soustrayant la première concentration ($c_1(x)$), la deuxième concentration ($c_2(x)$) et la quatrième concentration ($c_4(x)$) d'1.

7. Système tomodensitométrique selon la revendication 1,
dans lequel ledit jeu de données de projection d'effet photoélectrique et ledit jeu de données de projection d'effet de Compton dérivés par ladite unité de modélisation (104) sont des quantités indépendantes en énergie.

8. Système tomodensitométrique selon la revendication 3, dans lequel ledit au moins un composant est indiqué par un agent de contraste injecté dans ledit objet.

9. Système tomodensitométrique selon la revendication 8, dans lequel ledit agent de contraste est de l'iode ou du gadolinium.

10. Système tomodensitométrique selon la revendication 1 à 9, dans lequel au moins un desdits composants est représenté par un ou plusieurs sous-composants.

**11.** Système tomodensitométrique selon la revendication 10,
dans lequel lesdits coefficients d'atténuation de Compton et coefficients d'atténuation photoélectrique connus dudit au moins un composant sont obtenus par ladite unité de modélisation sous forme de dit jeu de données de projection d'effet photoélectrique et dit jeu de données de projection d'effet de Compton, respectivement.

**12.** Dispositif de traitement de données (110) destiné à être utilisé dans un système tomodensitométrique pour déterminer les concentrations relatives quantitatives de matériaux des composants dans une région d'intérêt d'un objet avec des coefficients d'atténuation de Compton et coefficients d'atténuation photoélectrique connus pour lesdits composants, des données de projection tomodensitométriques spectrales de ladite région d'intérêt étant fournies audit dispositif de traitement de données, lesdites données de projection tomodensitométriques spectrales étant obtenues à partir d'une unité tomographique (102), comprenant :

   - une unité de modélisation (104) pour obtenir un jeu de données de projection d'effet photoélectrique et un jeu de données de projection d'effet de Compton en décomposant ledit jeu de données de projection tomodensitométriques spectrales au moyen de modèles respectifs d'effet photoélectrique et d'effet de Compton ;
   - une unité de reconstruction (106) pour reconstruire une image à effet photoélectrique et une image à effet de Compton de ladite région d'intérêt à partir dudit jeu de données de projection d'effet photoélectrique et dudit jeu de données de projection d'effet de Compton ;
   - une unité de traitement (108) pour déterminer les concentrations desdits composants dans ladite région d'intérêt en résolvant un système d'équations obtenues en associant lesdites données d'image à effet photoélectrique avec les produits accumulés desdites concentrations et desdits coefficients d'atténuation photoélectrique pour lesdits composants et en associant lesdites données d'image à effet de Compton avec les produits accumulés desdites concentrations et desdits coefficients d'atténuation de Compton pour lesdits composants pour obtenir une première concentration ($c_1(x)$) d'un premier composant et une deuxième concentration ($c_2(x)$) d'un deuxième composant et en soustrayant la première concentration ($c_1(x)$) et la deuxième concentration ($c_2(x)$) d'1 pour obtenir une troisième concentration ($c_3(x)$) d'un troisième composant.

**13.** Procédé de traitement de données destiné à être utilisé dans un système tomodensitométrique pour déterminer les concentrations relatives quantitatives de matériaux des composants dans une région d'intérêt d'un objet avec des coefficients d'atténuation de Compton et coefficients d'atténuation photoélectrique connus pour lesdits composants, des données de projection tomodensitométriques de ladite région d'intérêt étant fournies audit procédé de traitement de données, lesdites données de projection tomodensitométriques étant obtenues à partir d'une unité tomographique, comprenant les étapes suivantes :

   - obtention (204) d'un jeu de données de projection d'effet photoélectrique et d'un jeu de données de projection d'effet de Compton en décomposant ledit jeu de données de projection tomodensitométriques spectrales au moyen de modèles respectifs d'effet photoélectrique et d'effet de Compton ;
   - reconstruction (206) d'une image à effet photoélectrique et d'une image à effet de Compton de ladite région d'intérêt à partir dudit jeu de données de projection d'effet photoélectrique et dudit jeu de données de projection d'effet de Compton ;
   - détermination (208) des concentrations desdits composants dans ladite région d'intérêt en résolvant un système d'équations obtenues en associant lesdites données d'image à effet photoélectrique avec les produits accumulés desdites concentrations et desdits coefficients d'atténuation photoélectrique pour lesdits composants et en associant lesdites données d'image à effet de Compton avec les produits accumulés desdites concentrations et desdits coefficients d'atténuation de Compton pour lesdits composants pour obtenir une première concentration ($c_1(x)$) d'un premier composant et une deuxième concentration ($c_2(x)$) d'un deuxième composant et en soustrayant la première concentration ($c_1(x)$) et la deuxième concentration ($c_2(x)$) d'1 pour obtenir une troisième concentration ($c_3(x)$) d'un troisième composant.

**14.** Programme d'ordinateur comprenant des moyens codes de programme d'ordinateur pour entraîner la réalisation, par un ordinateur, des étapes du procédé selon la revendication 13 lorsque ledit programme d'ordinateur est exécuté sur un ordinateur.

scanning unit ⌐102

10⌐ radiation source | detector unit ⌐12

modeling unit ⌐104

reconstruction unit ⌐106

processing unit ⌐108

data processing device ⌐110

# FIG. 1

```
                    ╭─────────╮
                    │  start  │
                    ╰────┬────╯
                         │
                         ▼
        ┌────────────────────────────────────┐
        │    aquisition of CT projection data │────202
        └────────────────┬───────────────────┘
                         │
                         ▼
        ┌────────────────────────────────────┐
        │  obtaining photoelectric effect     │
        │  data set and Compton effect data   │────204
        │  set                                │
        └────────────────┬───────────────────┘
                         │
                         ▼
        ┌────────────────────────────────────┐
        │  reconstructing a photoelectric     │
        │  effect image and a Compton effect  │────206
        │  image                              │
        └────────────────┬───────────────────┘
                         │
                         ▼
        ┌────────────────────────────────────┐
        │    determining concentrations of    │
        │       the components                │────208
        └────────────────┬───────────────────┘
                         │
                         ▼
                    ╭─────────╮
                    │   end   │
                    ╰─────────╯
```

# FIG. 2

Gadolinium

FIG. 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20040101088 A1

- US 20040264628 A1

### Non-patent literature cited in the description

- **KALENDER, W.A. et al.** Evaluation of a Prototype Dual Energy Computer Tomographic Apparatus. I. Phantom Studies. *Medical physics,* May 1986, vol. 13 (3), 334-339

- **P. SUKOVIC et al.** Basis Material Decomposition Using Triple-Energy X-ray Computer Tomography. *Proceedings of the 16th IEEE Instrumentation and Measurement Technology Conference, 1999,* 1999, vol. 3, ISBN 0-7803-5276-9, 1615-1618